# EUROPÄISCHE PATENTSCHRIFT

(11) **EP 1 183 367 B1**
(45) Veröffentlichungstag und Bekanntmachung des Hinweises auf die Patenterteilung: **27.12.2006**
(21) Anmeldenummer: 00936846.5
(22) Anmeldetag: 31.05.2000
(51) Int. Cl.: C12N 15/37, C07K 14/025, C12N 5/22, A61K 38/16, A61K 39/12, G01N 33/68, C12N 15/62

(54) **ZYTOTOXISCHES T-ZELLEPITOP DES PAPILLOMAVIRUS L1-PROTEINS UND SEINE VERWENDUNG IN DIAGNOSTIK UND THERAPIE**
CYTOTOXIC T-CELL EPITOPE OF THE PAPILLOMAVIRUS L1-PROTEIN AND USE THEREOF IN DIAGNOSTICS AND THERAPY
EPITOPE CYTOTOXIQUES DES LYMPHOCYTES T, DE PROTEINE L1 DU PAPILLOMAVIRUS, ET UTILISATION DANS LES DOMAINES DU DIAGNOSTIC ET DE LA THERAPIE

(30) Priorität: 01.06.1999 DE 19925235
(43) Veröffentlichungstag der Anmeldung: 06.03.2002
(73) Patentinhaber: MediGene AG, 82152 Planegg/Martinsried (DE); Deutsches Krebsforschungszentrum, 69120 Heidelberg (DE)
(72) Erfinder: JOCHMUS, Ingrid, 6230 Brixxlegg (AT); NIELAND, John, 8000Aaarhus (DK); OSEN, Wolfram, D-69121 Heidelberg (DE); FAATH, Stefan, 6230 Brixlegg (AT); SCHÄFER, Klaus, D-68623 Lampertheim (DE)
(74) Vertreter: Bösl, Raphael Konrad
(86) Internationale Anmeldenummer: PCT/EP2000/005005
(87) Internationale Veröffentlichungsnummer: WO 2000/073464

(56) Entgegenhaltungen:
- WO-A-93/02184
- WO-A-96/11272
- WO-A-99/18220
- GB-A- 2 279 651
- S -Y CHAN ET AL: "Phylogenetic analysis of 48 papillomavirus types and 28 subtypes and variants: a showcase of the molecular evolution of DNA viruses" JOURNAL OF VIROLOGY,US,NEW YORK, US, Bd. 66, Nr. 10, 1. Oktober 1992 (1992-10-01), Seiten 5714-5725, XP002094682 ISSN: 0022-538X

### SEQUENZPROTOKOLL

<110> MediGeneAG
<110> Deutsches Krebsforschungszentrum
<120> Zytotoxische T-Zellepitope des Papillomavirus L1-Proteins und ihre Verwendung in Diagnostik und Therapie
<150> 19925235.1
   <151> 1999-06-01
<160> 17
<170> MS Word 97/Windows NT
<210> 1
   <211> 9
   <212> PRT
   <213> Humanes Papillomavirus Typ 16
<400> 1 
Ala Gln Ile Phe Asn Lys Pro Tyr Trp 9
1
<210> 2
   <211> 9
   <212> PRT
   <213> Humanes Papillomavirus Typ 16
<400> 2 
Ala Gly Val Asp Asn Arg Glu Cys Ile 9
1
<210> 3
   <211> 9
   <212> PRT
   <213> Influenzavirus Typ A
<400> 3 
Ala Ser Asn Glu Asn Met Glu Thr Met 9
1
<210> 4
   <211> 8
   <212> PRT
   <213> Humanes Papillomavirus Typ 16
<400> 4 
Gly Ala Met Asp Phe Thr Thr Leu 8
1
<210> 5
   <211> 8
   <212> PRT
   <213> Humanes Papillomavirus Typ 16
<400> 5 
Gly Asp Ser Leu Phe Phe Tyr Leu 8
1
<210> 6
   <211> 8
   <212> PRT
   <213> Humanes Papillomavirus Typ 16
<400> 6 
Met Gln Val Thr Phe Ile Tyr Ile 8
1
<210> 7
   <211> 8
   <212> PRT
   <213> Humanes Papillomavirus Typ 16
<400> 7 
Val Tyr His Ile Phe Phe Gln Met 8
1
<210> 8
   <211> 8
   <212> PRT
   <213> Humanes Papillomavirus Typ 16
<400> 8 
Val His Thr Gly Phe Gly Ala Met 8
1
<210> 9
   <211> 8
   <212> PRT
   <213> Humanes Papillomavirus Typ 16
<400> 9 
Lys Tyr Pro Asp Tyr Ile Lys Met 8

1
<210> 10
   <211> 8
   <212> PRT
   <213> Humanes Papillomavirus Typ 16
<400> 10 
Val Thr Phe Ile Tyr Ile Leu Val 8
1
<210> 11
   <211> 8
   <212> PRT
   <213> Humanes Papillomavirus Typ 16
<400> 11 
Leu Glu Asp Thr Tyr Arg Phe Val 8

1
<210> 12
   <211> 8
   <212> PRT
   <213> Humanes Papillomavirus Typ 16
<400> 12 
Gly Asn Gln Leu Phe Val Thr Val 8
1
<210> 13
   <211> 8
   <212> PRT
   <213> Humanes Papillomavirus Typ 16
<400> 13 
Lys Lys Tyr Thr Phe Val Thr Val 8
1
<210> 14
   <211> 8
   <212> PRT
   <213> Humanes Papillomavirus Typ 16
<400> 14 
Glu Asn Asp Val Asn Tyr His Ile 8
1
<210> 15
   <211> 9
   <212> PRT
   <213> Humanes Papillomavirus Typ 16
<400> 15 
Ala Gly Val Asp Asn Arg Glu Cys Ile 9
1
<210> 16
   <211> 10
   <212> PRT
   <213> Humanes Papillomavirus Typ 16
<400> 16 
Thr Val Gly Glu Asn Val Pro Asp Asp Leu 10
1
<210> 17
   <211> 10
   <212> PRT
   <213> Humanes Papillomavirus Typ 16
<400> 17

Tyr Lys Asn Thr Asn Phe Lys Glu Tyr Leu 10
1

## Beschreibung

Die vorliegende Erfindung betrifft ein Papillomavirus T-Zell-Epitop mit einer Aminosäuresequenz, AGVDNRECI, und/oder eine funktionell aktive Variante davon, sowie ihre Verwendung in Diagnostik und Therapie.

Die Papillomviren, auch Warzenviren genannt, sind doppelsträngige DNA-Viren mit einer Genomgröße von etwa 8000 Basenpaaren und einem Ikosaederförmigen Kapsid mit einem Durchmesser von ca. 55 nm. Bis heute sind mehr als 100 verschiedene humanpathogene Papillomavirustypen (HPV) bekannt, von denen einige, z.B. HPV-16, HPV-18, HPV-31, HPV-33, HPV-39, HPV-45, HPV-52 oder HPV-58, bösartige Tumore und andere, z.B. HPV-6, HPV-11 oder HPV-42 gutartige Tumore verursachen können.

Das Genom der Papillomaviren läßt sich in drei Bereiche unterteilen: Der erste Bereich betrifft eine nicht-kodierende Region, die Regulationselemente für die Transkription und Replikation des Virus enthält. Die zweite Region, sogenannte E-(Early)Region enthält verschiedene Protein-kodierende Abschnitte E1-E7, von denen z.B. das E6- und das E7-Protein für die Transformation von Epithelzellen verantwortlich sind und das E1-Protein die DNA-Kopienzahl kontrolliert. Bei der E6- und E7-Region handelt es sich um sogenannte Onkogene, die auch in bösartig entarteten Zellen exprimiert werden. Die dritte Region, auch L-(Late)Region genannt, enthält zwei Protein-kodierende Abschnitte L1 und L2, die für Strukturkomponenten des Viruskapsids kodieren. Das L1-Protein ist zu über 90% im viralen Kapsid vorhanden, wobei das Verhältnis von L1:L2 im allgemeinen 30:1 ist. Unter dem Begriff L1-Protein versteht man im Sinne der vorliegenden Erfindung das Hauptkapsid Protein der Papillomaviren (Baker T. et al. (1991) Biophys. J. 60, 1445).

In über 50% der Fälle wird HPV-16 mit Gebärmutterhalskrebs (Cervixcarcinom) in Verbindung gebracht. HPV-16 ist der Hauptrisikofaktor für die Ausbildung von cervicalen Neoplasien. Das Immunsystem spielt eine wichtige Rolle beim Fortschreiten der Krankheit. So sind vermutlich zelluläre Immunantworten und insbesondere Antigen-spezifische T-Lymphozyten wichtig für den Abwehrmechanismus. Es wurde weiterhin gefunden, daß in hochgradig malignen cervicalen intraepithelialen Neoplasien (CIN II/III) und cervicalen Tumoren das E7-Gen konstitutiv in allen Schichten des infizierten Epithels exprimiert wird. Daher wird vor allem das E7-Protein als potentielles Tumorantigen und als Zielmolekül für aktivierte T-Zellen betrachtet (siehe z.B. WO 93/20844). Die E7-induzierte zelluläre Immunantwort im Patienten ist aber anscheinend nicht stark genug, um den Krankheitsverlauf zu beeinflussen. Die Immunantwort kann eventuell durch geeignete Impfstoffe verstärkt werden.

Es konnte gezeigt werden, daß die Expression des L1-Gens bzw. die Coexpression des L1- und L2-Gens zur Bildung von Capsomeren, stabilen Capsomeren, Capsiden oder Virus-ähnliche Partikel (VLPs für virus-like particle) führen kann (siehe z.B. WO 93/02184, WO 94/20137 oder WO 94/05792). Unter Capsomeren versteht man eine oligomere Konfiguration, die aus fünf L1-Proteinen aufgebaut ist. Das Capsomer ist der Grundbaustein, aus denen virale Capside aufgebaut sind. Unter stabilen Capsomeren versteht man Capsomere, die nicht dazu in der Lage sind sich zu Capsiden zusammenzusetzen. Unter Capsiden versteht man die Hülle des Papillomavirus, die beispielsweise aus 72 Capsomeren zusammengesetzt ist (Baker T. et al. (1991) Biophys. J. 60, 1445). Unter VLP versteht man ein Capsid, das morphologisch und in seiner Antigenität einem intakten Virus gleicht. Die VLPs konnten zur Auslösung einer humoralen Immunantwort, die durch Bildung von neutralisierenden Antikörpern charakterisiert ist, in verschiedenen tierischen Systemen verwendet werden. Die Bildung von virus-neutralisierenden Antikörpern gegen L1- und/oder L2-Protein ist jedoch von geringerer klinischer Bedeutung, wenn die Virusinfektion bereits stattgefunden hat, da für die Eliminierung virus-infizierter Zellen keine Antikörper, sondern eine virus-spezifische zytotoxische T-Zell-(CTL)-Antwort notwendig zu sein scheint. Und obwohl VLPs in der Lage sind eine zytotoxische T-Zell-Antwort auszulösen, scheint eine ausschließlich gegen die Kapsidproteine L1 und/oder L2 gerichtete Immunantwort nicht zur Bekämpfung eines durch Papillomaviren bedingten Tumors geeignet.

Es wurden daher sogenannte chimäre Papillomavirus-ähnliche Partikel (CVLPs für chimeric virus-like particle) entwickelt, die aus einem Fusionsprotein des Kapsidproteins L1 und des potentiellen Tumorantigen E7 bestehen (WO 96/11272 und Müller, M. et al. (1997) Virology, 234, 93). Die CVLPs lösten nur im geringen Maße eine gegen das E7-Protein gerichtete humorale Immunantwort aus (Müller, M. et al. (1997), supra). Einige der getesteten CVLPs induzieren jedoch tatsächlich die erwünschte E7-spezifische zytotoxische T-Zellantwort in Mäusen (siehe auch Peng S. et al. (1998) Virology 240,147-57). CVLPs sind dadurch sowohl für die Entwicklung eines Impfstoffes als auch für die Behandlung bereits bestehender Infektionen und daraus resultierender Tumore interessant, da die über MHC-Moleküle der Klasse I-präsentierten E7-Peptide von Tumorzellen Zielmoleküle von zytotoxischen T-Zellen darstellen würden.

Einem Impfstoff bestehend aus CVLPs liegt das Prinzip der Pseudoinfektion der Zellen durch die CVLPs zugrunde. Dies bedeutet, daß die CVLPs wie Viren in die Zelle gelangen, dort zu Peptiden prozessiert werden, die Peptide auf MHC-Klasse I und II-Moleküle geladen werden und letztendlich CD8- bzw. CD4-positiven T-Zellen präsentiert werden. CD8-Zellen können als Folge dieser Stimulation zu zytotoxischen T-Zellen differenzieren und dann eine zelluläre Immunantwort bewirken, CD4-Zellen hingegen entwickeln sich zu T-Helferzellen und stimulieren B-Zellen zu einer humoralen oder CD8-positive T-Zellen zu einer zytotoxischen Immunantwort und können selbst die Lyse von infizierten Zellen induzieren.

Kleine Peptide können bereits auf der Zelloberfläche an MHC-Klasse I-Moleküle binden und dann ohne weitere Prozessierung CD8- oder CD4-positive Zellen zu einer zellulären Immunantwort stimulieren. Ein bestimmtes Peptid kann jedoch nur durch bestimmte MHC-Moleküle gebunden werden. Durch den großen Polymorphismus der MHC-Moleküle in natürlichen Populationen kann deshalb ein bestimmtes Peptid lediglich durch einen kleinen Teil einer Population gebunden und präsentiert werden. Unter Präsentation im Sinne der vorliegenden Erfindung wird verstanden, wenn ein Peptid oder Proteinfragment an ein MHC-Molekül bindet, wobei diese Bindung beispielsweise im endoplasmatischen Retikulum, im extrazellulären Raum, den Endosomen, Proendosomen, Lysosomen oder Protysosomen, stattfinden kann, und wenn dann dieser MHC-Molekül-Peptid-Komplex auf der extrazellulären Seite der Zellmembran gebunden ist, so daß er durch Immunzellen spezifisch erkannt werden kann.

Da CVLPs sowohl eine zelluläre als auch humorale Immunantwort auslösen und nicht MHC-restringiert sind, eignet sich diese Technologie generell zur Entwicklung von Impfstoffen, indem die Fähigkeit zur Partikelbildung durch einen L1-Anteil zur Verfügung gestellt wird und ein zusätzlicher Antigenanteil an diesen L1-Anteil fusioniert wird.

Bei der Entwicklung derartiger CVLPs ist es unbedingt notwendig, ein funktionelles Testsystem zur Verfügung zu haben, mit dem man direkt die Immunogenität von CVLPs untersuchen kann. Ein derartiges Testsystem sollte die Eigenschaft besitzen, daß CVLPs mit unterschiedlichen Antigenanteilen mit demselben Testsystem untersucht werden können. Da für immunologische Therapieverfahren von Tumoren oder Viruserkrankungen die zelluläre Immunantwort von entscheidender Bedeutung ist, stellte sich die Aufgabe, die durch CVLPs hervorgerufene zelluläre Immunantwort meßbar zu machen.

Die Lösung dieser Aufgabe gelang durch die Identifikation von T-Zell-Epitopen, die in Verbindung mit MHC-Molekülen, in einer besonderen Ausführungsform mit (H2-D^{b}) MHC-Molekülen in vivo und in vitro beispielsweise eine zytotoxische T-Zellantwort auslösen. Diese Peptide haben vorzugsweise die Sequenz AQIFNKPYW oder AGVDNRECI. Diese Sequenzen sind Bestandteil des L1-Peptid des HPV16. Sie umfassen die Aminosäurebereiche 330 bis 338 (L1₃₃₀₋₃₃₈) bzw. 165 bis 173 (L1₁₆₅₋₁₇₃)·

Die vorliegende Erfindung betrifft daher ein T-Zell-Epitop mit einer Aminosäuresequenz AGVDNRECI.

Unter einer funktionell aktiven Variante von AGVDNRECI versteht man ein T-Zell-Epitop, das in einem T-Zell-Zytotoxizitäts-Testsystem (siehe beispielsweise Beispiele 2-5 der vorliegenden Erfindung) eine, an der Zytotoxizität von AGVDNRECI gemessene Zytotoxizität besitzt, die mindestens der Summe aus dem Mittelwert der Negativkontrollen und der dreifachen Standardabweichung entspricht, vorzugsweise von mindestens ca. 30%, insbesondere mindestens ca. 50% und in besonders bevorzugter Weise von mindestens ca. 80%.

Eine bevorzugte Variante ist beispielsweise ein T-Zell-Epitop mit einer Sequenzhomologie zu AGVDNRECI von mindestens ca. 65%, vorzugsweise mindestens ca. 75% und insbesondere mindestens ca. 85% auf Aminosäureebene. Solche Epitope können aufgefunden werden, indem man gegen das T-Zell-Epitop AGVDNRECI spezifische T-Zellen generiert (DeBruijn M.L. et al. (1991) Eur. J. Immunol. 21, 2963-70; und DeBruijn M.L. (1992) Eur. J. Immunol. 22, 3013-20) und beispielsweise synthetisch hergestellte Peptide nach Wahl auf Erkennung durch die peptidspezifischen T-Zellen getestet werden (siehe Beispiele). Insbesondere werden unter T-Zellepitopen zytotoxische T-Zellepitope verstanden. Es sind jedoch auch nicht zytotoxische T-Zellen bekannt, die ebenfalls MHC I-Moleküle erkennen können, so daß auch nicht-zytotoxische T-Zellepitope als Variante von der vorliegenden Erfindung umfaßt sind.

Um die Verbindung zu detektieren oder in ihrer Aktivität der Bindung an T-Zellen zu modifizieren, kann sie eine chemische, radioaktive Isotopen-, nicht radioaktive Isotopen-, und/oder Fluoreszensmarkierung des T-Zell-Epitops und/oder des genannten Fusionsproteins enthalten.

Beispiele von dem Fachmann bekannten chemischen Substanzen, die sich für eine erfindungsgemäße chemische Markierung eignen, sind: Biotin, FITC (Fluoresceinisothiocyanat) oder Streptavidin.

Eine mögliche Ausführungsform ist, daß ein Peptid derart modifiziert wird, daß es mindestens ein Lysin enthält. An dieses Lysin wird in der dem Fachmann bekannter Weise Biotin oder FITC (fluorescin isothiocyanat) gekoppelt. Ein derartig modifiziertes Peptid wird an ein entsprechendes MHC-Molekül oder an eine Zelle mit entsprechenden MHC-Molekülen gebunden. Daraufhin kann das Peptid über markiertes Avidin oder Streptavidin bzw. direkt über die Fluoreszenz des FITC nachgewiesen werden.

Beispiele von dem Fachmann bekannten Isotopen, die sich für eine erfindungsgemäße radioaktive Isotopenmarkierung eignen, sind: ³H, ¹²⁵I, ¹³¹I, ³²P, ³³P oder ¹⁴C.

Beispiele von dem Fachmann bekannten Isotopen, die sich für eine erfindungsgemäße nicht radioaktive Isotopenmarkierung eignen, sind: ²H oder ¹³C.

Beispiele von dem Fachmann bekannten fluoreszierenden Substanzen, die sich für eine erfindungsgemäße Fluoreszensmarkierung eignen, sind: ¹⁵²Eu, Fluoresceinisothiocyanat, Rhodamin, Phycoerythrin, Phycocyanin, Allophycocyanin, o-Phtaldehyd oder Fluorescamin.

Dem Fachmann sind weitere hier nicht aufgeführte Markierung bekannt, die auch im Sinne dieser Erfindung zur Markierung eingesetzt werden können.

Beispiele von dem Fachmann bekannten erfindungsgemäßen chemischen Modifikationen sind die Übertragung von Acetyl-, Phosphat-, und/oder Monosacharidgruppen

Polypeptide mit einer Aminosäurelänge von ca. 50 können beispielsweise durch eine chemische Peptidsynthese hergestellt werden. Längere Polypetide werden vorzugsweise gentechnisch erzeugt. Ein weiterer Gegenstand der vorliegenden Erfindung ist daher ein Nukleinsäurekonstrukt zur Expression des genannten T-Zell-Epitops, das folgende Komponenten enthält: (a) mindestens ein regulatorisches Element und (b) mindestens eine Nukleinsäure, die für eine Aminosäuresequenz der Verbindung kodiert. Das genannte Nukleinsäurekonstrukt ist vorzugsweise aus DNA oder RNA. Geeignete regulatorische Elemente erlauben beispielsweise die konstitutive, regulierbare, gewebsspezifische, zellzyklusspezifische oder metabolischspezifische Expression in eukaryotischen Zellen oder die konstitutive, metabolischspezifische oder regulierbare Expression in prokaryotischen Zellen. Regulierbare Elemente gemäß der vorliegenden Erfindung sind Promotoren, Aktivatorsequenzen, Enhancer, Silencer, und/oder Repressorsequenzen.

Beispiele für geeignete regulierbare Elemente, die konstitutive Expression in Eukaryoten ermöglichen sind Promotoren die von der RNA Polymerase III erkannt werden oder virale Promotoren, wie CMV-Enhancer, CMV-Promotor, SV40 Promotor und virale Promotor-und Aktivatorsequenzen, abgeleitet aus beispielsweise HBV, HCV, HSV, HPV, EBV HTLV oder HIV.

Beispiele für regulierbare Elemente, die regulierbare Expression in Eukaryoten ermöglichen sind der Tetrazyklinoperator in Kombination mit einem entsprechenden Repressor (Gossen M. et al (1994) Curr. Opin. Biotechnol. 5, 516-20).

Beispiele für regulierbare Elemente, die gewebsspezifische Expression in Eukaryoten ermöglichen sind Promotoren oder Aktivatorsequenzen aus Promotoren oder Enhancern von solchen Genen, die für Proteine kodieren, die nur in bestimmten Zelltypen exprimiert werden.

Beispiele für regulierbare Elemente, die zellzyklusspezifische Expression in Eukaryoten ermöglichen sind die Promotoren der folgenden Gene: cdc25C, Cyclin A, Cyclin E, cdc2, E2F, B-myb oder DHFR (Zwicker J. und Müller R. (1997) Trends Genet. 13, 3-6).

Beispiele für regulierbare Elemente, die metabolisch spezifische Expression in Eukaryoten ermöglichen sind Promotoren, die durch Hypoxie, durch Glucosemangel, durch Phosphatkonzentration oder durch Hitzeschock reguliert werden.

Um die Einführung der genannten Nukleinsäure und damit die Expression des Polypeptids in einer eu- oder prokaryotischen Zelle durch Transfektion, Transformation oder Infektion zu ermöglichen, kann die Nukleinsäure als Plasmid, als Teil eines viralen, oder nicht viralen Vektors vorliegen. Ein weiterer Gegenstand der vorliegenden Erfindung ist deshalb ein Vektor, insbesondere ein Expressionsvektor, der eine Nukleinsäure codierend für ein erfindungsgemäßes Polypeptid enthält. Als virale Vektoren eignen sich hierbei besonders: Baculoviren, Vakziniaviren, Adenoviren, adenoassoziierte Viren und Herpesviren. Als nicht virale Vektoren eignen sich hierbei besonders: Virosomen, Liposomen, kationische Lipide, oder Poly-Lysin konjugierte DNA.

Ein weiterer Gegenstand der vorliegenden Erfindung ist eine Zelle, die das T-Zell Epitop mit einer Aminosäuresequenz AGVDNRECI enthält, vorzugsweise präsentiert. In einer besonderen Ausführungsform wird die Zelle durch einen der genannten Vektoren transfiziert, transformiert oder infiziert. Diese Zelle exprimiert unter dem Fachmann bekannten Bedingungen, die zur Aktivierung der jeweils verwendeten regulierbaren Elemente fuhren, das erfindungsgemäße Polypeptid. Das Polypeptid kann dann aus dieser Zelle isoliert und z.B. unter Verwendung einer der oben genannten Markierungen aufgereinigt werden. Zur gentechnischen Herstellung und anschließenden Aufreinigung der exprimierten Verbindungen eignen sich prokaryotische und eukaryotische Zellen, insbesondere Bakterienzellen wie beispielsweise E. coli, Hefezellen wie beispielsweise S. cerevisiae, Insekten Zellen wie beispielsweise Spodoptera frugiperda Zellen (Sf-9) oder Trichoplusia ni Zellen oder Säugerzellen wie beispielsweise COS-Zellen oder HeLa-Zellen.

Eine bevorzugte Ausführungsform ist es, die Zelle, die das erfindungsgemäße Polypeptide exprimiert, selbst zu verwenden, wobei in einer besonders bevorzugten Ausführungsform die Zelle Teile das erfindungsgemäßen Polypeptids über MHC-1 Moleküle auf der Zelloberfläche präsentiert. Für die Herstellung einer erfindungsgemäßen Zelle sind Antigen-präsentierende Zellen wie beispielsweise B-Zellen, Makrophagen, Dendritischen Zellen, nicht menschlichen embryonalen Zellen oder Fibroblasten, in einer bevorzugten Ausführungsform B16F10-, B6-, C3-, EL4-, RMA-oder RMA-S-Zellen geeignet. Die erfindungsgemäßen Zellen, die ein Polypeptid enthaltend ein T-Zell-Epitop, präsentieren, können als Zielzellen zur Restimulation von Immunzellen, insbesondere T-Zellen und/oder zur Messung der Aktivierung von T-Zellen eingesetzt werden. Unter Zielzelle im Sinne der vorliegenden Erfindung ist eine Zelle zu verstehen, die über MHC-Moleküle ein T-Zell-Epitope präsentiert und damit spezifisch eine T-Zell-Aktivierung hervorruft, insbesondere eine zytotoxische T-Zeil-Reaktion gegen die Zelle.

Ferner kann die ein T-Zell-Epitop enthaltene Verbindung, Teil eines Komplexes sein, der dadurch charakterisiert ist, daß die Verbindung kovalent oder über hydrophobe Wechselwirkungen, Ionenbindung oder Wasserstoffbrückenbindungen mit mindestens einer weiteren Spezies wie Peptide, Proteine, Peptoide, lineare oder verzweigte Oligo- oder Polysacharide und Nukleinsäuren verbunden ist.

Gegenstand der vorliegenden Erfindung ist daher ein Komplex enthaltend das T-Zell-Epitop AGVDNRECI und mindestens eine weitere Verbindung. Eine bevorzugte Ausführungsform ist, daß das Polypeptid in Verbindung mit MHC Klasse I-Molekülen, vorzugsweise als H2-D^{b}-Tetramer vorliegt. Besonders bevorzugt sind humane oder murine MHC-Klasse I Moleküle, insbesondere ein von C57B1/6 Mäusen abgeleitetes MHC-Klasse I Molekül. Durch die Technik von Altman J.D. et al. (1996, Science 274, 94-6) lassen sich beispielsweise H2-D^{b}-Tetramere mit den entsprechenden gebundenen Peptiden herstellen, die in der Lage sind, an die T-Zellrezeptoren von peptidspezifischen zytotoxischen T-Zellen zu binden.

Eine weitere Ausführungsform ist die Immobilisierung der Verbindung oder des genannten Komplexes an Trägermaterialien. Als Trägermaterialien eignen sich beispielsweise Keramik, Metall, insbesondere Edelmetall, Gläser, Kunststoffe, kristalline Materialien bzw. dünne Schichten des Trägers, insbesondere der genannten Materialien, oder (bio)molekulare Filamente wie Cellulose oder Gerüstproteine.

Zur Aufreinigung des erfindungsgemäßen Komplexes kann eine Komponente des Komplexes zusätzlich noch ein Protein-Tag enthalten. Erfindungsgemäße Protein-Tags erlauben beispielsweise die hochaffine Absorption an eine Matrix, stringentes Waschen mit geeigneten Puffern ohne den Komplex im nennenswerten Maße zu eluieren und anschließende gezielte Elution des absorbierten Komplexes. Beispiele von dem Fachmann bekannten Protein-Tags, sind ein N- oder C-terminales (HIS)₆-Tag, ein Myc-Tag, ein FLAG-Tag, ein Hämaglutenin-Tag, Glutathion-Transferase (GST)-Tag, Intein with an Affinity Chitin-binding Tag oder Maltose binding protein (MBP)-Tag. Die erfindungsgemäßen Protein-Tags können sich N-, C-terminal, und/oder intern befinden.

Ein anderer Gegenstand der vorliegenden Erfindung ist ein Verfahren zum in vitro Nachweis der Aktivierung von T-Zellen durch mindestens eine Verbindung, enthaltend ein T-Zell-Epitop. Ein derartiges Verfahren besteht vorzugsweise aus drei Schritten:
a) In einem ersten Schritt werden Zellen mit mindestens einer Verbindungen enthaltend ein T-Zell-Epitop stimuliert. Diese Verbindung kann mindestens eine Verbindung enthaltend ein erfindungsgemäße, T-Zell-Epitop, mindestens einen erfindungsgemäßen Komplex enthaltend ein T-Zell-Epitop, mindestens ein Capsomer, mindestens ein stabiles Capsomer, mindestens ein VLP, mindestens ein CVLP, und/oder mindestens ein Virus bedeuten. In einer bevorzugten Ausführungsform werden Immunzellen durch die Inkubation mit CVLPs stimuliert. Diese Stimulation kann beispielsweise in Form einer Impfung erfolgen, oder durch Inkubation von Immunzellen mit CVLPs in vitro. Derartig stimulierte Immunzellen werden beispielsweise nach einer Impfung aus der Milz, aus Lymphknoten oder aus dem Blut gewonnen, und/oder werden kultiviert.
b) In einem zweiten Schritt werden Zellen mit mindestens einem erfindungsgemäßen T-Zell-Epitop, mindestens einer Verbindung enthaltend ein erfindungsgemäßes T-Zell-Epitop, mindestens einer Zielzelle, die ein T-Zell-Epitop präsentiert und/oder mit mindestens einem erfindungsgemäßen Komplex inkubiert.
c) In einem dritten Schritt erfolgt die Bestimmung der Aktivierung von T-Zellen. Hierfür geeignete Verfahren sind beispielsweise der Nachweis der Produktion oder Sekretion von Cytokinen durch die T-Zellen, der Expression von Oberflächenmolekülen auf T-Zellen, der Lyse von Zielzellen oder der Proliferation von Zellen. Hierfür geeignete Methoden sind beispielsweise ein Cytokinassay (Chapter 6.2 bis 6.24 in Current Protocols in Immunology (1999), edited by Coligan J.E., Kruisbeek A.M., Margulies D.H., Shevach E.M. and Strober W., John Wiley & Sons), ELISPOT (Chapter 6.19 in Current Protocols in Immunology, supra), ein ⁵¹Cr-Freisetzungstest (Chapter 3.11 in Current Protocols in Immunology, supra) oder der Nachweis der Proliferation (Chapter 3.12 in Current Protocols in Immunology, supra). Je nach verwendeter Methode kann dabei auch zwischen den Immunzellen wie zytotoxischen T-Zellen, T-Helferzellen, B-Zellen, NK-Zellen und anderen Zellen unterschieden werden. Die Verwendung von Verbindungen, Komplexen, und/oder Zellen, die erfindungsgemäße Markierungen enthalten, erlaubt die Detektion von T-Zellen, die das T-Zell-Epitop erkennen über den Nachweis der Bindung markierter Verbindungen, Komplexe, und/oder Zellen an die T-Zellen. In einer bevorzugten Ausführungsform wird die Bindung erfindungsgemäßer MHC-Polypeptid-Komplexe an die Oberfläche der T-Zellen nachgewiesen. Dies kann derart durchgeführt werden, daß die MHC-Komplexe selbst markiert, beispielsweise fluoreszenzmarkiert sind, oder daß man in einem weiteren Schritt einen MHC-spezifischen, markierten, beispielsweise fluoreszenzmarkierten Antikörper verwendet, um wiederum die MHC-Komplexe nachzuweisen. Die Fluoreszenzmarkierung der T-Zellen läßt sich dann beispielsweise in einem 'Fluorescens Activated Cell Sorter' (FACS) messen und auswerten. Eine andere Möglichkeit zum Nachweis der Bindung von den Komplexen an die T-Zellen ist erneut die Messung der Aktivierung von T-Zellen (Cytokinassay, Elispot, ⁵¹Cr-Freisetzungstest, Proliferation, siehe oben). Allerdings benötigt man hierfür die gleichzeitige Stimulation von Korezeptoren (z. B. CD28), beispielsweise durch Korezeptor-spezifische Antikörper (Anti-CD28) und/oder andere unspezifische Aktivatoren (IL-2).

Gegenstand der vorliegenden Erfindung ist auch ein Verfahren, das einen zusätzlichen Schritt a') enthält, der nach Schritt a) eingeführt wird.
a') In diesem zusätzlichen dem Schritt a) nachfolgenden Schritt a') werden werden die isolierten oder kultivierten Zellen mit mindestens einer Zielzelle beladen mit einer Verbindung enthaltend ein erfindungsgemäßen T-Zell-Epitop, mindestens einem erfindungsgemäßen Komplex enthaltend ein T-Zell-Epitop, mindestens einem Capsomer, mindestens einem stabilen Capsomer, mindestens einem VLP, mindestens einem CVLP und/oder mindestens einem Virus, mit mindestens einem erfindungsgemäßen Komplex enthaltend ein T-Zell-Epitop, und/oder mindestens einer Zielzelle, die ein T-Zell-Epitop präsentiert, für mindestens ca. 12 Tage, insbesondere ca. 5 Tage cokultiviert, bevor sich Schritt b) anschließt. Unter Cokultivierung ist das Wachstum der Zellen:
   (i) in Gegenwart von mindestens einer Zielzelle beladen mit einer Verbindung enthaltend ein erfindungsgemäßes T-Zell-Epitop, mindestens einem erfindungsgemäßen Komplex enthaltend ein T-Zell-Epitop, mindestens einem Capsomer, mindestens einem stabilen Capsomer, mindestens einem VLP, mindestens einem CVLP, und/oder mindestens einem Virus,
   (ii) in Gegenwart mindestens eines erfindungsgemäßen Komplexes enthaltend ein T-Zell-Epitop,
   (iii) in Gegenwart mindestens einer Zielzelle, die ein T-Zell-Epitop präsentiert,
im selben Wachstumsmedium und selben Gewebekulturbehälter zu verstehen.

Ein weiterer Gegenstand der vorliegenden Erfindung ist ein Verfahren zur Herstellung einer T-Zell-Epitop präsentierenden Zielzelle. Hierbei ist es möglich, die Zielzelle mit Kombinationen von unterschiedlichen T-Zell-Epitopen zu beladen. In einer bevorzugten Ausführungsform wird die Zielzelle mit mindestens einer Verbindung enthaltend ein T-Zell-Epitop und/oder mindestens einem Komplex enthaltend ein T-Zell-Epitop inkubiert. In einer besonders bevorzugten Ausführungsform wird die Zielzelle in Wachstumsmedium, das erfindungsgemäße Polypeptide enthält, oder mit MHC Klasse I-Komplexen mit gebundenen erfindungsgemäßen Polypeptiden inkubiert. Die MHC Klasse I-Komplexe können beispielsweise als H2-D^{b}-Tetramere vorliegen. Ein Tetramer bindet dabei in der Regel vier Peptide. Diese können sowohl identisch sein oder aber unterschiedliche Spezies von Peptiden repräsentieren. In einer weiteren bevorzugten Ausführungsform wird die Zielzelle mit einer Nukleinsäure und/oder einem Vektor transfiziert, transformiert und/oder infiziert. In einer besonders bevorzugten Ausführungsform wird die Zielzelle mit einem Vakziniavirusvektor infiziert. Das erfindungsgemäße Verfahren wird mit Antigen-präsentierenden Zellen beispielsweise mit B-Zellen, Makrophagen, Dendritischen Zellen, nicht menschlichen embryonalen Zellen oder Fibroblasten, in einer bevorzugten Ausführungsform mit B16F10-, B6-, C3-, EL4-, RMA- oder RMA-S-Zellen durchgeführt.

Die verwendeten CVLPs enthalten ein Papillomavirus L1-Protein oder Varianten davon, insbesondere HPV16 L1-Protein und, jedoch nicht notwendigerweise, ein zu L1 heterologes Protein oder Varianten davon. Die zwei Proteine können direkt oder indirekt gebunden vorliegen. Direkt gebunden meint im Sinne der Erfindung, daß eine kovalente Bindung zwischen den beiden Proteinen vorliegt, beispielsweise eine Peptidbindung oder eine Disulfidbindung. Indirekt gebunden bedeutet, daß die Proteine über nicht-kovalente Bindungen gebunden sind, beispielsweise hydrophobe Wechselwirkungen, Ionenbindungen oder Wasserstoffbrücken. In einer weiteren Ausführungsform enthalten die CVLPs zusätzlich zu L1-Protein oder Varianten davon ein Papillomavirus L2-Protein.

Eine bevorzugte Ausführung des L1-Proteins der vorliegenden Erfindung sind beispielsweise L1-Proteine mit einer oder mehreren Deletionen, insbesondere einer C-terminalen Deletion. Eine C-terminale Deletion hat den Vorteil, daß die Effizienz der Bildung virus-ähnlicher Partikel gesteigert werden kann, da das am C-Terminus lokalisierte nukleäre Lokalisationssignal deletiert wird. Die C-terminale Deletion beträgt daher vorzugsweise bis zu ca. 35 Aminosäuren, insbesondere ca. 25 bis ca. 35 Aminosäuren, vor allem ca. 32 bis ca. 34 Aminosäuren. Beispielsweise ist eine 32 Aminosäuren lange C-terminale Deletion des HPV16 L1-Proteins ausreichend, um die Bildung von virus-ähnlichen Partikeln um das mindestens ca. zehnfache steigern zu können. Des weiteren kann das L1-Protein eine oder mehrere Mutationen tragen oder der L1-Anteil aus L1-Proteinen verschiedener Papillomaviren zusammengesetzt sein. Gemeinsames Kennzeichen der erfindungsgemäßen L1-Proteine ist, daß sie die Bildung von VLPs bzw. CVLPs zulassen und daß sie mindestens ein erfindungsgemäßes T-Zell-Epitop enthalten.

In einer bevorzugten Ausführungsform ist das L1-Protein oder Varianten davon und das zu L1 heterologe Protein ein Fusionsprotein. Beinhaltet sind auch heterologe Proteine, die aus mehreren verschiedenen Proteinen oder Teilen davon zusammengesetzt sind. Dies können beispielsweise auch Epitope, insbesondere zytotoxische T-Zellepitope von Proteinen sein. Epitope im Sinne der Erfindung können dabei auch Teil eines synthetischen Polypeptids mit einer Länge von ca. 50 Aminosäuren, vorzugsweise von mindestens ca. 35 Aminosäuren, insbesondere von mindestens ca. 20 Aminosäuren und in besonders bevorzugter Weise von mindestens ca. 9 Aminosäuren sein.

Bevorzugt sind zu L1 heterologe Proteine, die von einem viralen Protein abgeleitet sind, beispielsweise abgeleitet von HIV, HBV oder HCV, vorzugsweise von Papillomaviren, insbesondere von humanen Papillomaviren.

In einer bevorzugten Ausführungsform ist dies ein E-Protein eines Papillomavirus, vorzugsweise ein E6- und/oder E7-Protein. Insbesondere ist bevorzugt, wenn das E-Protein ein deletiertes E-Protein ist, vorzugsweise ein C-terminale deletiertes, insbesondere ein C-terminal deletiertes E7-Protein, da diese Konstrukte in Verbindung mit deletiertem L1-Protein bevorzugt virus-ähnliche Partikel ausbilden können. Insbesondere bevorzugt sind Deletionen bis zu 55 Aminosäuren, vorzugsweise ca. 5 bis ca. 55 Aminosäuren, insbesondere ca. 38 bis ca. 55 Aminosäuren.

In einer weiteren Ausführung kann das zu L1 heterologe Protein von Antigenen nicht viraler Erreger abstammen. Ebenso können sie von Autoimmunantigenen wie z.B. Thyroglobulin, Myelin basic protein oder Zona Pellucida Glycoprotein 3 (ZP₃), die mit bestimmten Autoimmunkrankheiten wie z.B. Thyroiditis, Multiple Sklerose, Oophoritis oder rheumatoider Arthritis assoziiert sind, abgeleitet sein. In einer bevorzugten Ausführung stammt das zu L1 heterologe Protein von Tumorantigenen ab, vorzugsweise Melanomaantigenen wie MART, Ovarialcarcinomantigenen wie Her2 neu (c-erbB2), BCRA-1 oder CA125, Coloncarcinomantigenen wie CA125 oder Mammacarcinomantigenen wie Her2 neu (c-erbB2), BCRA-1, BCRA-2.

Ein weiterer Gegenstand dieser Erfindung ist ein Verfahren zum in vitro Nachweis der Aktivierung von T-Zellen, die durch Präparation aus Proben gewonnen werden. Dieses Verfahren erlaubt es zu bestimmen, ob in einer Probe, beispielsweise einer Blutprobe eines Patienten oder in der Milz einer Maus, Papillomavirus L1-Protein-spezifische zytotoxische T-Zellen vorhanden sind. Ein derartiges Nachweisverfahren enthält die folgenden Schritte:
a") In einem ersten Schritt werden Zellen gewonnen, beispielsweise durch Blutabnahme von einem Patienten oder durch die Präparation zum Beispiel der Milz oder von Lymphknoten einer Maus. Anschließend werden die Zellen in Wachstumsmedium aufgenommen und kultiviert.
b) In einen zweiten Schritt werden Zellen mit mindestens einer Zielzelle, die ein T-Zell-Epitop präsentiert oder mit mindestens einem Komplex, der als eine Komponente eine Verbindung enthaltend ein T-Zell-Epitope umfaßt, inkubiert.
c) In einem dritten Schritt erfolgt die Bestimmung der Aktivierung von T-Zellen. Hierfür geeignete Verfahren sind beispielsweise der Nachweis der Produktion oder Sekretion von Cytokinen durch die T-Zellen. der Expression von Oberflächenmolekülen auf T-Zellen, der Lyse von Zielzellen oder der Proliferation von Zellen. Hierfür geeignete Methoden sind beispielsweise ein Cytokinassay (Chapter 6.2 bis 6.24 in Current Protocols in Immunology (1999), edited by Coligan J.E., Kruisbeek A.M., Margulies D.H., Shevach E.M. and Strober W., John Wiley & Sons), ELISPOT (Chapter 6.19 in Current Protocols in Immunology, supra), ein ⁵¹Cr-Freisetzungstest (Chapter 3.11 in Current Protocols in Immunology, supra) oder der Nachweis der Proliferation (Chapter 3.12 in Current Protocols in Immunology, supra). Je nach verwendeter Methode kann dabei auch zwischen den Immunzellen wie zytotoxischen T-Zellen, T-Helferzellen, B-Zellen, NK-Zellen und anderen Zellen unterschieden werden. Die Verwendung von Verbindungen, Komplexen, und/oder Zellen die erfindungsgemäße Markierungen enthalten erlaubt die Detektion von T-Zellen, die das T-Zell-Epitop erkennen über den Nachweis der Bindung markierter Verbindungen, Komplexe, und/oder Zellen an die T-Zellen. In einer bevorzugten Ausführungsform wird die Bindung erfindungsgemäßer MHC-Polypeptid-Komplexe an die Oberfläche der T-Zellen nachgewiesen. Dies kann derart durchgeführt werden, daß die MHC-Komplexe selbst markiert, beispielsweise fluoreszenzmarkiert sind, oder daß man in einem weiteren Schritt einen MHC-spezifischen, markierten, beispielsweise fluoreszenzmarkierten Antikörper verwendet, um wiederum die MHC-Komplexe nachzuweisen. Die Fluoreszenzmarkierung der T-Zellen läßt sich dann beispielsweise in einem 'Fluorescens Activated Cell Sorter' (FACS) messen und auswerten. Eine andere Möglichkeit zum Nachweis der Bindung von den Komplexen an die T-Zellen ist erneut die Messung der Aktivierung von T-Zellen (Cytokinassay, Elispot, ⁵¹Cr-Freisetzungstest, Proliferation, siehe oben). Allerdings benötigt man hierfür die gleichzeitige Stimulation von Korezeptoren (z. B. CD28), beispielsweise durch Korezeptor-spezifische Antikörper (Anti-CD28) und/oder andere unspezifische Aktivatoren (IL-2).

Gegenstand der vorliegenden Erfindung ist auch ein Verfahren, das einen zusätzlichen Schritt a') enthält, der nach Schritt a") eingeführt wird.
a') In diesem zusätzlichen dem Schritt a") nachfolgenden Schritt a') werden die isolierten oder kultivierten Zellen mit mindestens einer Zielzelle beladen mit einer Verbindung enthaltend ein erfindungsgemäßen T-Zell-Epitop, mindestens einem erfindungsgemäßen Komplex enthaltend ein T-Zell-Epitop, mindestens einem Capsomer, mindestens einem stabilen Capsomer, mindestens einem VLP, mindestens einem CVLP und/oder mindestens einem Virus, mit mindestens einem erfindungsgemäßen Komplex enthaltend ein T-Zell-Epitop, und/oder mindestens einer Zielzelle, die ein T-Zell-Epitop präsentiert, für mindestens ca. 12 Tage, insbesondere ca. 5 Tage cokultiviert, bevor sich Schritt b) anschließt. Unter Cokultivierung ist das Wachstum der Zellen:
   (i) in Gegenwart von mindestens einer Zielzelle beladen mit einer Verbindung enthaltend ein erfindungsgemäßes T-Zell-Epitop, mindestens einem erfindungsgemäßen Komplex enthaltend ein T-Zell-Epitop, mindestens einem Capsomer, mindestens einem stabilen Capsomer, mindestens einem VLP, mindestens einem CVLP, und/oder mindestens einem Virus,
   (ii) in Gegenwart mindestens eines erfindungsgemäßen Komplexes enthaltend ein T-Zell-Epitop,
   (iii) in Gegenwart mindestens einer Zielzelle, die ein T-Zell-Epitop präsentiert,
im selben Wachstumsmedium und selben Gewebekulturbehälter zu verstehen.

Ein weiterer Gegenstand der Erfindung ist ein Testsystem (Kit) zum in vitro Nachweis der Aktivierung von T-Zellen enthaltend:
a) mindestens ein erfindungsgemäßes T-Zell-Epitop, mindestens einen erfindungsgemäßen Vektor, mindestens eine erfindungsgemäße Zelle, und/oder mindestens einen erfindungsgemäßen Komplex, und
b) Effektorzellen des Immunsystems, vorzugsweise T-Zellen, insbesondere zytotoxische T-Zellen oder T-Helferzellen.

Ein weiterer Gegenstand der Erfindung ist ein Testsystem (Kit) zum in vitro Nachweis der Aktivierung von T-Zellen enthaltend:
a) mindestens ein erfindungsgemäßes T-Zell-Epitop, mindestens einen erfindungsgemäßen Vektor, mindestens eine erfindungsgemäße Zelle, und/oder mindestens einen erfindungsgemäßen Komplex, und
b) Effektorzellen des Immunsystems, vorzugsweise T-Zellen, insbesondere zytotoxische T-Zellen oder T-Helferzellen.

Das Testsystem wird in einer besonderen Ausführungsform zur Bestimmung der, beispielsweise in einer Blutprobe eines Patienten oder in der Milz einer Maus vorhandenen L1-Protein-spezifischen zytotoxischen T-Zellen verwendet. In diesem Fall sind die in b) beschriebenen Zellen im Testsystem enthaltene Kontroll-zellen, deren Aktivierung durch die erste Kitkomponente, die unter a) genannten Substanzen, als Standard dient. Die in dieser Reaktion beobachteten Aktivierung wird mit die T-Zell-Aktivierung von aus Patienten oder Mäusen isolierten Zellen durch die Kitkomponente a) verglichen.

In einer weiteren besonderen Ausführungsform wird das Testsystem beispielsweise zur Bestimmung der L1-Protein-spezifischen Antigenität einer Verbindung enthaltend ein T-Zell-Epitop, eines Komplexes enthaltend ein T-Zell-Epitop, eines Capsomers, eines stabilen Capsomers, eines VLPs, eines CVLPs, und/oder eines Virus verwendet. In diesem Fall sind die in a) beschriebenen Substanzen Kontrollsubstanzen deren aktivierende Wirkung auf die zweite Kitkomponente, die unter b) genannten Zellen, als Standard dient. Die in dieser Reaktion beobachteten Aktivierung wird mit der aktivierenden Wirkung einer Verbindung enthaltend ein T-Zell-Epitop, einem Komplex enthaltend ein T-Zell-Epitop, einem Capsomer, einem stabilen Capsomer, einem VLP, ein CVLP, und/oder einem Virus auf die Kitkomponente b) verglichen.

Des weiteren betrifft die Erfindung die Verwendung von mindestens einem T-Zell-Epitop, mindestens einem erfindungsgemäßen Vektor enthaltend eine Nukleinsäure kodierend für eine ein T-Zell-Epitop enthaltende Verbindung, mindestens einer erfindungsgemäßen Zelle enthaltend ein T-Zell-Epitop zur, und/oder mindestens einem erfindungsgemäßen Komplex enthaltend ein T-Zell-Epitop zur Auslösung oder zum Nachweis einer Immunantwort.

Zur Stimulation von Immunzellen in vitro wie in vivo eignen sich insbesondere Zellen, die mindestens eines der erfindungsgemäßen Moleküle über ihre MHC-Klasse I-Moleküle präsentieren. Zur Antigen-Präsentation geeignete Zellen sind z. B. B-Zellen, dendritische Zellen, Macrophagen, nicht menschliche embryonale Zellen oder Fibroblasten, die durch eine gemeinsame Kultivierung mit Immunzellen eine Stimulation von spezifischen T-Zellen erreichen können.

In einer besonderen Ausführungsform kann eine erfindungsgemäße Verbindung beispielsweise ein HPV18 L1E7-Fusionsprotein, das zusätzlich ein erfindungsgemäßes T-Zell-Epitop enthält, zur Detektion einer Immunantwort eingesetzt werden. Eine solche erfindungsgemäße Verbindung kann die Fähigkeit zur Bildung von CVLPs besitzen.

Ein weiterer Gegenstand der Erfindung ist ein Arzneimittel oder Diagnostikum enthaltend mindestens eine erfindungsgemäße Verbindung enthaltend ein T-Zell-Epitop, mindestens einen Vektor enthaltend eine Nukleinsäure kodierend für eine ein T-Zell-Epitop enthaltende Verbindung, mindestens eine erfindungsgemäße Zelle enthaltend ein T-Zell-Epitop, und/oder mindestens einen erfindungsgemä-ßen Komplex enthaltend ein T-Zell-Epitop und gegebenenfalls einen pharmazeutisch akzeptablen Träger.

Beispiele von dem Fachmann bekannten Trägern sind Glas, Polystyren, Polypropylen, Polyethylen, Dextran, Nylon, Amylase, natürliche oder modifizierte Zellulose, Polyacrylamide, Agarose, Aluminiumhydroxid oder Magnitid.

Ein erfindungsgemäßes Arzneimittel oder Diagnostikum kann in Lösung vorliegen, an eine feste Matrix gebunden sein und/oder mit einem Adjuvans versetzt sein.

Das Arzneimittel oder Diagnostikum kann auf verschiedene Weisen verabreicht werden. Beispiele von dem Fachmann bekannten Verabreichungsformen sind parenterale, lokale und/oder systemische Applikation durch z. B. orale, intranasale, intravenöse, intramuskuläre, und/oder topische Applikation. Die bevorzugte Applikationsform wird beispielsweise durch den natürlichen Infektionsweg der jeweiligen Papillomavirusinfektion beeinflußt. Die verabreichte Menge richtet sich nach Alter, Gewicht, allgemeinem Gesundheitszustand des Patienten und dem Typ der Papillomavirusinfektion. Das Arzneimittel oder Diagnostikum kann in Form von Kapseln, Lösung, Suspension, Elixier (für orale Applikation) oder sterile Lösungen bzw. Suspensionen (für parenterale oder intranasale Applikation) verabreicht werden. Als inerter und immunologisch akzeptabler Träger kann beispielsweise Salzlösung oder phosphatgepufferte Salzlösung verwendet werden. Das Arzneimittel wird in therapeutisch effektiven Mengen verabreicht. Das bedeutet Mengen, die ausreichend sind, um eine schützende immunologische Antwort hervorzurufen.

In einer besonderen Ausführungsform kann eine Verbindung beispielsweise ein HPV18 L1E7-Fusionsprotein, das zusätzlich ein erfindungsgemäßes T-Zell-Epitop enthält, als Arzneimittel oder Diagnostikum eingesetzt werden. Eine solche Verbindung kann die Fähigkeit zur Bildung von CVLPs besitzen.

Die Figuren und die folgenden Beispiele sollen die Erfindung näher erläutern.

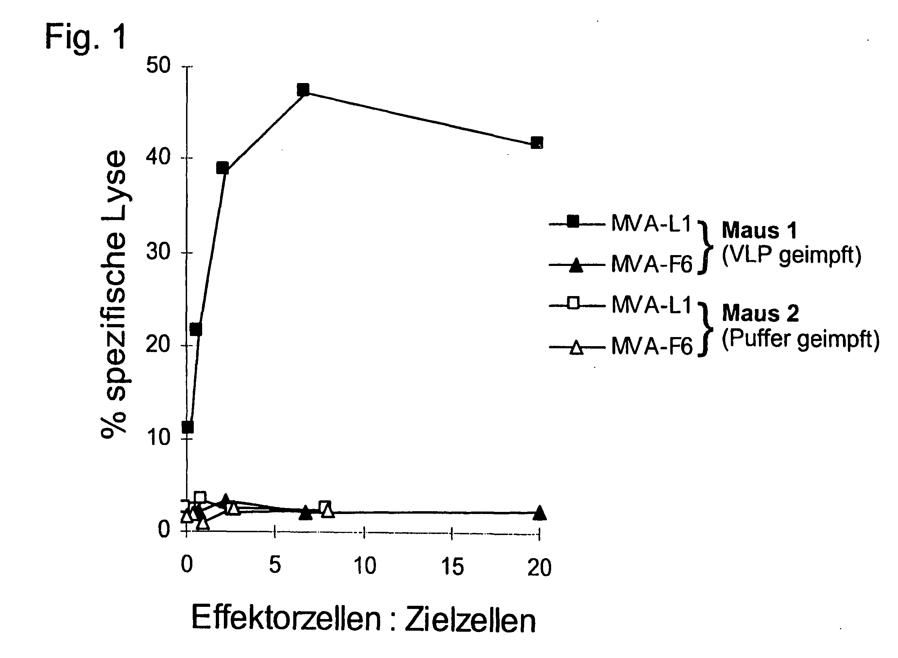

Fig. 1 zeigt die graphische Auswertung der spezifischen Lyse von verschiedenen murinen B6-Zellen, die entweder mit MVA-L1_{ΔC} oder mit MVA-F6 infiziert worden waren, durch Milzzellen von Mäusen, die entweder mit VLPs bzw. mit Puffer geimpft worden waren. Aufgetragen ist das Verhältnis der Effektorzellen zu den Zielzellen gegen die Prozent der spezifisch lysierten Zellen.

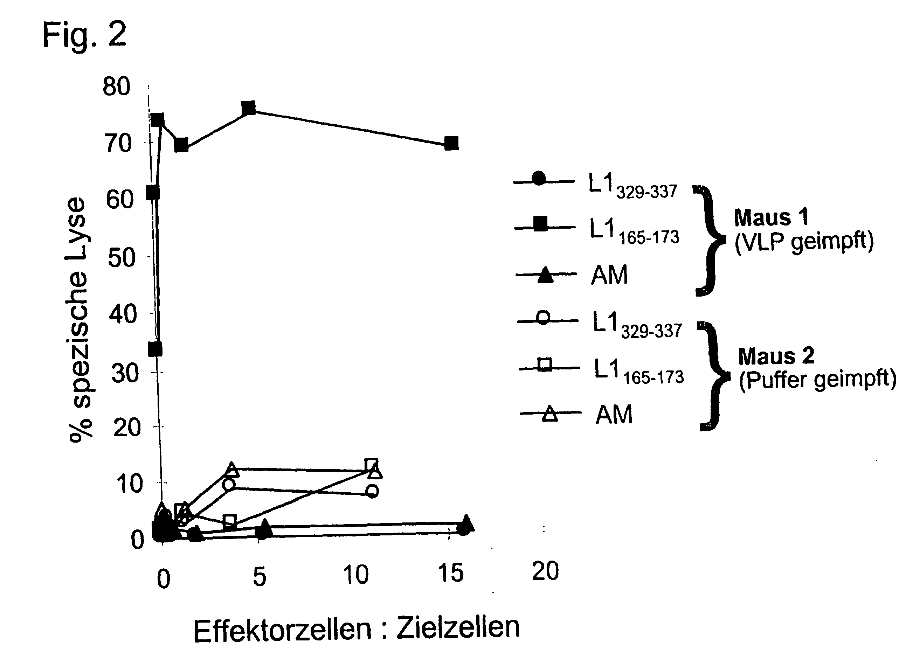

Fig. 2 zeigt die graphische Auswertung der spezifischen Lyse von verschiedenen RMA-S-Zellen, die entweder L1₃₃₀₋₃₃₈, L1₁₆₅₋₁₇₃ oder AM präsentieren, durch die Milzzellen von Mäusen, die mit VLPs bzw. mit Puffer geimpft worden waren. Aufgetragen ist das Verhältnis der Effektorzellen zu den Zielzellen gegen die Prozent der spezifisch lysierten Zellen.

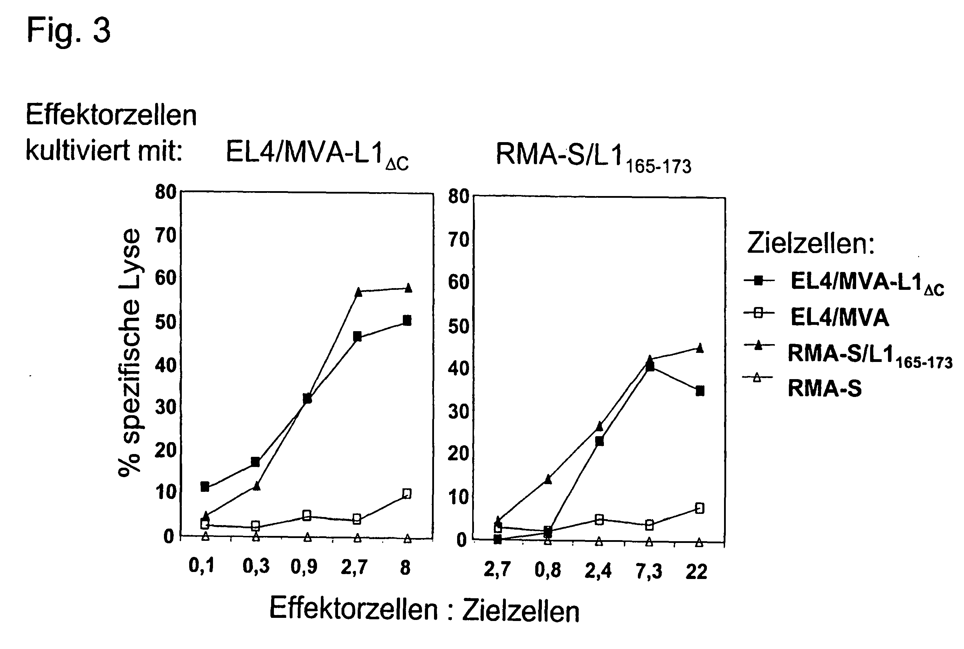

Fig. 3 zeigt die graphische Auswertung der spezifischen Lyse von vier verschiedenen Zellinien durch T-Zellen, die zuvor mit MVA-L1_{ΔC}-infizierten EL4-Zellen (links) oder mit L1₁₆₅₋₁₇₃-beladenen RMA-S-Zellen restimuliert worden waren. Aufgetragen ist das Verhältnis der Effektorzellen zu den Zielzellen gegen die Prozent der spezifisch lysierten Zellen.

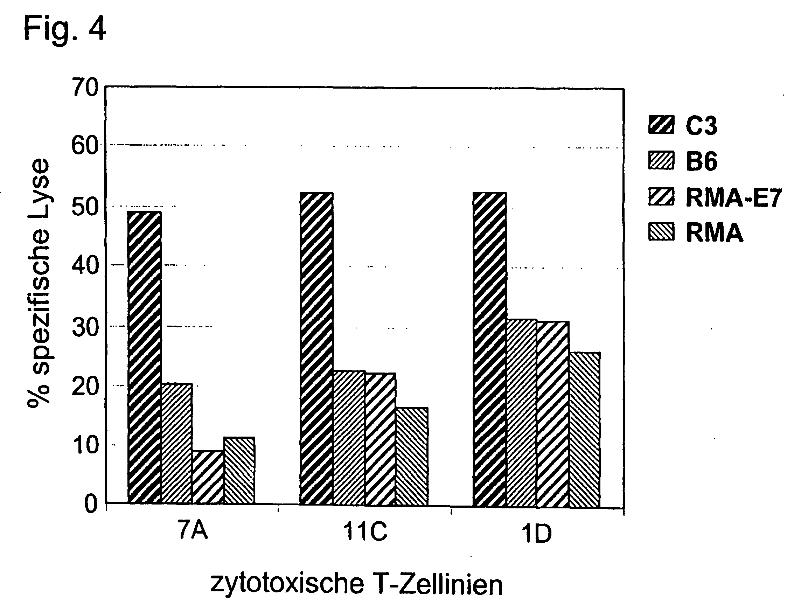

Fig. 4 zeigt die graphische Auswertung der spezifischen Lyse von vier verschiedenen Zielzellinien durch drei verschiedene zytotoxische T-Zellinien.

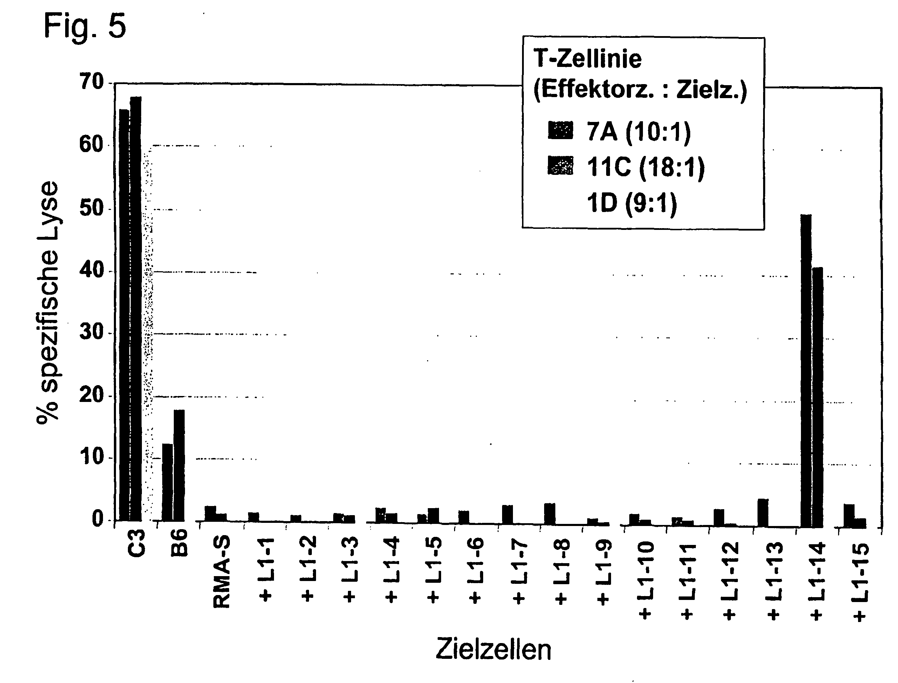

Fig. 5 zeigt die graphische Auswertung der spezifischen Lyse von unterschiedlichen Zielzellen, entweder verschiedenen Zellinien oder RMA-S-Zellen, die mit den L1-Peptiden L1-1 bis L1-15 beladen worden waren, durch drei verschiedene zytotoxische T-Zellinien.

### Beispiele

### 1. Beschreibung der Ausgangsmaterialien

- Die Herstellung von HPV16 L1_{ΔC}E7₁₋₅₅ CVLPs erfolgte gemäß der deutschen Patentanmeldung DE 198 12 941.6 (siehe auch Müller M. et al. (1997) Virology 234, 93-111).
- L1 VLPs (siehe Müller M. et al. (1997) Virology 234, 93-111)
- C57B1/6-Mäuse wurden von Charles River Laboratories (Wilmington, MA, USA) bezogen.
- MVA-L1_{ΔC} bedeutet rekombinantes murines Vakziniavirus, das in infizierten Zellen HPV16 L1_{ΔC} exprimiert.
- MVA-F6 bedeutet Vakziniavirus (Kontrollvirus).
- B6-Zellen bedeutet embyonale Stammzellen aus einer C57B 1/6-Maus.
- C3-Zellen bedeutet HPV16 und ras-transformierte B6-Embryozellen (siehe Feltkamp M.C. et al. (1993) Eur. J. Immunol. 23, 2242-9).
- RMA-Zellen stammen aus einem Thymom einer C57BL/6-Maus (siehe Ljunggren H.G. & Kärre K. (1985) J. Exp. Med. 162, 1745-59).
- RMA-S-Zellen stammen aus einem Thymom einer C57BL/6-Maus (siehe Ljunggren H.G. & Kärre K. (1985) J. Exp. Med. 162, 1745-59). Sie weisen einen Defekt im Antigenprozessierungs-assoziiertem Transport auf, der zur Unterbindung der Beladung von MHC-1 Molekühlen im endoplasmatischen Retikulum führt. Die dennoch auf der Zelloberfläche vorhandenen unbeladenen MHC-1 Moleküle können beispielsweise durch Inkubation der Zellen in Peptid-haltigen Medien beladen werden, so daß sich diese Zellen sehr gut zur Präsentation eines Antigens eignen (siehe Powis S.J. et al. (1991) Nature 354, 528-31).
- EL4-Zellen stammt aus einem Thymom einer C57B1/6-Maus (siehe Shevach E.M. et al. (1972) J. Immunol. 108, 1146-51), z. B. ATCC TIB-49.
- Zellen wurden jeweils bei 37°C und 5% CO₂ in RPMI-Medium (Gibco BRL, Eggenstein) mit 10% fötalem Kälberserum, Kanamycin, Ampicillin kultiviert.
- AM-Peptid bedeutet Aminosäuren 366 bis 374 des Influenza Nukleoproteins, Sequenz: ASNENMETM (siehe Townsend A.R. et al. (1986) Cell 44, 959-68).
- L1-Peptide bedeutet von HPV16 abgeleitetes Peptid des L1 Hauptkapsidproteins.

### 2. Induktion von L1-spezifischen CTL nach Immunisierung mit L1 VLPs

a) Immunisierung von Mäusen mit VLPs:
   Zwei C57B1/6-Mäuse wurden mit 10 µg L1 VLPs (Maus 1) oder zur Kontrolle mit Puffer (Maus 2) immunisiert. Nach 6 Wochen wurden die Milzzellen isoliert.
b) Herstellung von Antigen-präsentierenden Zellen (Zielzellen):
   B6-Zellen wurden für 2,5 Tage mit Interferon y inkubiert, anschließend über Nacht mit MVA-L1_{ΔC}-Viren (MOI = 5) für die Herstellung Antigen-präsentierender Zellen oder mit MVA-F6 für die Herstellung von Kontroll-zellen infiziert und für 16h kultiviert. In einem nächsten Schritt wurden die infizierten B6-Zellen bestrahlt und dadurch am weiteren Wachstum gehindert.
c) Restimulation der isolierten Milzzellen:
   Die Milzzellen aus Maus 1 und 2 wurden jeweils gemeinsam mit den L1_{ΔC}-exprimierenden B6-Zellen, die als Stimulatorzellen für die T-Zellen der Milzzellen fungierten, für 5 Tage kultiviert.
d) Test der Zytotoxizität der isolierten Milzzellen:
   Die stimulierten Milzzellen (Effektorzellen) der Maus 1 (VLP-geimpft) und 2 (Puffer-geimpft) wurden entweder mit MVA-L1_{ΔC}-infizierten oder mit MVA-F6-infizierten B6-Zellen (Zielzellen), die jeweils zuvor in Anwesenheit von ⁵¹Cr für 1h bei 37°C inkubiert worden waren, in fünf verschiedenen Verhältnissen von Effektorzellen zu Zielzellen für 4h inkubiert. Die spezifische Lyse der Zielzellen wurde durch die Freisetzung des radioaktiven ⁵¹Cr in einem β-Counter gemessen (siehe Fig. 1).

Ergebnis: Die Milzzellen der L1-immunisierten Maus lysierten die MVA-L1_{ΔC}-infizierten Zielzellen, die MVA-F6-infizierten Zielzellen hingegen nicht. Die Milzzellen besaßen somit eine spezifische Zytotoxizität gegen das L1-Protein. Die Kontroll-Milzzellen, die aus der mit Puffer geimpften Maus stammten, zeigten keine lytische Aktivität gegenüber den Zielzellen.

### 3. Peptid-spezifische Lyse von Zielzellen durch L1-spezifische T-Zellen (VLPgeimpfte Mäuse)

a) Herstellung Antigen-präsentierender Zellen:
   RMA-S-Zellen wurden während einer einstündigen Inkubation mit ⁵¹Cr bei 37°C zusätzlich mit einem Peptid (Konzentration 50 µM) inkubiert. Als Peptid wurde entweder
      • L1₃₃₀₋₃₃₈,
      • L1₁₆₅₋₁₇₃ oder
      • das AM-Peptid (als Kontrolle)
   verwendet.
b) Test der Zytotoxizität der isolierten Milzzellen:
   Die gemäß Beispiel 1c hergestellten stimulierten Milzzellen (Effektorzellen) der Maus 1 (VLP-geimpft) und 2 (Puffer-geimpft) wurden mit den Peptidinkubierten RMA-S-Zellen (Zielzellen) in Anwesenheit von 0,5 µg/ml des jeweiligen Peptids für 4h in sechs verschiedenen Verhältnissen von Effektorzellen zu Zielzellen inkubiert. Die spezifische Lyse der Zielzellen wurde durch die Freisetzung des radioaktiven ⁵¹Cr in einem β-Counter gemessen (siehe Fig. 2).

Ergebnis: Die mit L1₁₆₅₋₁₇₃ präinkubierten Zielzellen konnten durch die Milzzellen der L1-immunisierten Maus 1 effektiv lysiert werden, während die Präinkubation von RMA-S Zellen mit L1₃₃₀₋₃₃₈ oder dem Kontroll-Peptid AM keine deutliche Lyse der RMA-S Zellen durch die stimulierten Milzzellen bewirkte. Die Milzzellen der Puffer-geimpften Maus 2 zeigten für keine der Peptid präinkubierten RMA-S Zellen spezifische lytische Aktivität. Das Peptid L1₁₆₅₋₁₇₃ stellt somit in diesen Zellen ein spezifisches zytotoxisches T-Zellepitop dar.

### 4. Peptid-spezifische Lyse von Zielzellen durch L1-spezifischen T-Zellen (CVLPgeimpfte Maus)

a) Immunisierung von Mäusen mit CVLPs:
   Eine Maus wurde mit 10 µg L1_{ΔC}E7₁₋₅₅ CVLPs immunisiert. Die Milzzellen wurden nach 2 Wochen isoliert.
b) Herstellung von Antigen-präsentierenden Zellen:
   EL4-Zellen wurden wie in 1b) beschrieben mit MVA-L1_{ΔC}-infiziert. RMA-S-Zellen wurden wie in 3a) beschrieben mit L1₁₆₅₋₁₇₃-beladenen.
c) Restimulation der isolierten Milzzellen:
   Die isolierten Milzzellen von 4a) wurden jeweils für 12 Tage mit den beiden unter 4b) beschriebenen Zelltypen EL4/MVA-L1_{ΔC} bzw. RMA-S/L1₁₆₅₋₁₇₃-inkubiert.
d) Test der Zytotoxizität der isolierten Milzzellen
   Anschließend wurde ein Zytotoxizitätstest analog zu Beispiel 2d) oder 3b) durchgeführt. Dabei wurde jeweils die Lyse der EL4/MVA-L1_{ΔC}-Zellen bzw. der RMA-S/L1₁₆₅₋₁₇₃-Zellen in fünf verschiedenen Verhältnissen von Effektorzellen zu Zielzellen bestimmt. Als Kontrolle dienten entweder EL4-Zellen, die mit einem leeren MVA-F6-Vektor infiziert worden waren, bzw. RMA-S-Zellen, die nicht mit L1₁₆₅₋₁₇₃ vorinkubiert worden waren (siehe Fig. 3)

Ergebnis: Fig. 3 zeigt die Auswertung des ⁵¹Cr-Freisetzungstest analog zu Beispiel 1 oder 2. Die Milzzellen waren sowohl nach Kultivierung mit EL4/MVA-L1_{ΔC}-Zellen wie auch mit RMA-S/L1 ₁₆₅₋₁₇₃-Zellen in der Lage, die das L1-Peptid-präsentierenden Zielzellen effektiv zu lysieren (EL4/MVA-L1_{ΔC}-Zellen oder RMA-S/L1₁₆₅₋₁₇₃-Zellen), während die Kontroll-Zellen nicht lysiert wurden. Dadurch daß die Erkennung von L1-exprimierenden Zellen vergleichbar ist mit den Peptid-präsentierenden Zellen kann man folgern, daß das Peptid L1₁₆₅₋₁₇₃ im wesentlichen für die Induktion der T-Zellantwort verantwortlich ist.

Dieses Ergebnis macht erneut deutlich, daß in CVLP-geimpften Mäusen eine CD8-T-Zellantwort gegen das HPV 16 Peptid L1₁₆₅₋₁₇₃ induziert wird. Dabei ist es unwesentlich, ob die Impfung der Mäuse mit VLPs (siehe Beispiel 3) oder CVLPs (dieses Beispiel) durchgeführt wurde.

### 5. L1-spezifische zytotoxische T-Zellen

Eine Maus wurde zweimal mit 10⁷ C3-Zellen geimpft (diese sind durch HPV16 und ras transformiert). Anschließend wurden die Milzzellen isoliert und in Gegenwart von bestrahlten C3-Zellen als Stimulatorzellen kultiviert und durch Zugabe von C3-Zellen restimuliert. Durch Vereinzelung der Milzzellen wurden "Milzzellklone" kultiviert. Diese Klone wurden dann in Zytotoxizitätstests getestet, wie effizient sie C3-Zellen lysieren. In dem selben Test lysierten diese Klone weit weniger effizient B6, RMA- oder RMA-E7-Zellen (C3-Zellen exprimieren durch die HPV16-Transformation auch E7). Fig. 4 zeigt drei dieser zytotoxischen Klone (= T-Zellinien), die C3-Zellen deutlich effizienter lysieren als B6-, RMA-E7- oder RMA-Zellen.

Diese drei T-Zellinien wurden dann in einem Zytotoxiszitätstest gemäß einem der vorangegangenen Beispiele auf ihre Fähigkeit zur Lyse von C3-Zellen, von B6-Zellen, von RMA-S-Zellen sowie von RMA-S-Zellen, die zuvor mit verschiedenen L1-Peptiden (L1-1 bis L1-15; jeweilige Konzentration 50 µM) beladen worden waren, getestet. Fig. 5 zeigt, daß die T-Zellinien 7A und 11C sehr effizient L1-14-beladene RMA-S-Zellen lysieren können. Somit sind genannte T-Zellinien spezifisch für das L1-14-Peptid, bei dem es sich um das L1-Peptid 330-338 (L1₃₃₀₋₃₃₈) handelt. Die anderen getesteten Peptide wurden von diesen T-Zellinien nicht erkannt. Die Sequenzen der getesteten Peptide sind wie folgt: L1-1: GAMDFTTL, L1-2: GDSLFFYL, L1-3: MQVTFIYI, L1-4: VYHIFFQM, L1-5. VHTGFGAM, L1-6: KYPDYIKM, L1-7: VTFIYILV, L1-8: LEDTYRFV, L1-9: GNQLFVTV, L1-10: KKYTFVTV, L1-11: ENDVNYHI, L1-12: AGVDNRECI, L1-13: TVGENVPDDL, L1-14: AQIFNKPYW, L1-15: YKNTNFKEYL

## Patentansprüche

1. T-Zell-Epitop mit einer Aminosäuresequenz AGVDNRECI.

2. T-Zell-Epitop nach Anspruch 1, **dadurch gekennzeichnet, daß** das T-Zell-Epitop ein zytotoxisches T-Zell-Epitop ist.

3. Nukleinsäure, **dadurch gekennzeichnet, daß** sie für ein T-Zell-Epitop gemäß Anspruch 1 oder 2 kodiert.

4. Vektor, insbesondere ein Expressionsvektor, **dadurch gekennzeichnet, daß** er eine Nukleinsäure gemäß Anspruch 3 enthält.

5. Zelle, **dadurch gekennzeichnet, daß** sie mindestens ein T-Zell-Epitop gemäß Anspruch 1 oder 2 enthält, vorzugsweise präsentiert.

6. Zelle nach Anspruch 5, **dadurch gekennzeichnet, daß** die Zelle mit einer Nukleinsäure gemäß Anspruch 3 und/oder einem Vektor gemäß Anspruch 4 transfiziert, transformiert, und/oder infiziert ist.

7. Zelle nach Anspruch 5, **dadurch gekennzeichnet, daß** die Zelle mit mindestens einem T-Zell-Epitop gemäß Anspruch 1 oder 2 und/oder mindestens einem Komplex gemäß einem der Ansprüche 9-11 inkubiert wurde.

8. Zelle nach Anspruch 5 oder 6, **dadurch gekennzeichnet, daß** die Zelle eine B-Zelle, ein Makrophage, eine Dendritische Zelle, eine nicht menschliche embryonale Zelle, ein Fibroblast, eine B16F10-, eine B6-, eine C3-, eine EL4-, eine RMA-oder eine RMA-S-Zelle ist.

9. Komplex enthaltend ein T-Zell-Epitop gemäß Anspruch 1 oder 2 und mindestens eine weitere Verbindung.

10. Komplex nach Anspruch 9, **dadurch gekennzeichnet, daß** der Komplex mindestens ein MHC-Klasse I-Molekül, vorzugsweise als H2-D^{b}-Tetramer enthält.

11. Komplex nach Anspruch 10, **dadurch gekennzeichnet, daß** das genannte MHC-Klasse I Molekül ein humanes oder murines MHC-Klasse I Molekül, insbesondere ein von C57B1/6 Mäusen abgeleitetes MHC-Klasse I Molekül ist.

12. Verfahren zum in vitro Nachweis der Aktivierung von T-Zellen durch mindestens eine Verbindung, enthaltend ein T-Zell-Epitop gemäß Anspruch 1 oder 2, das folgende Schritte enthält:
a) Stimulation von Zellen mit mindestens einer genannten Verbindung;
b) Zugabe von mindestens einer Zielzelle, die ein T-Zell-Epitop gemäß Anspruch 1 oder 2 präsentiert oder eines Komplexes gemäß einem der Ansprüche 9-11, und
c) Bestimmung der Aktivierung von T-Zellen.

13. Verfahren nach Anspruch 12, **dadurch gekennzeichnet, daß** es nach Schritt
a) folgenden zusätzlichen Schritt a') enthält:
a') Cokultivierung der Zellen für ca. 12 Tage, insbesondere mindestens 5 Tage mit:
(i) mindestens einer Zielzelle beladen mit einem T-Zell-Epitop gemäß Anspruch 1 oder 2, mindestens einem Komplex gemäß einem der Ansprüche 9-11, mindestens einem Capsomer, mindestens einem stabilen Capsomer, mindestens einem VLP, mindestens einem CVLP, und/oder mindestens einem Virus,
(ii) mindestens einem Komplex gemäß einem der Ansprüche 9-11,
(iii) und/oder mindestens einer Zielzelle, die ein T-Zell-Epitop gemäß Anspruch 1 präsentiert,
bevor sich Schritt b) anschließt.

14. Verfahren zur Herstellung einer Zielzelle nach einem der Ansprüche 5, 7 oder 8, **dadurch gekennzeichnet, daß** die Zielzelle mit mindestens einem T-Zell-Epitop gemäß Anspruch 1 oder 2 und/oder mindestens einem Komplex gemäß einem der Ansprüche 9-11 enthaltend ein T-Zell-Epitop gemäß Anspruch 1 oder 2, inkubiert wird.

15. Verfahren zur Herstellung einer Zielzelle nach einem der Ansprüche 5, 7 oder 8, **dadurch gekennzeichnet, daß** die Zielzellen mit einer Nukleinsäure gemäß Anspruch 3 und/oder einem Vektor gemäß Anspruch 4 transfiziert, transformiert und/oder infiziert wird.

16. Verfahren zur Herstellung einer Zielzelle nach Anspruch 14 oder 15, **dadurch gekennzeichnet, daß** die Zielzelle eine B-Zelle, ein Makrophage, eine Dendritische Zelle, eine nicht menschliche embryonale Zelle, ein Fibroblast, eine B16F10-, eine B6-, eine C3-, eine EL4-, eine RMA- oder eine RMA-S-Zelle ist.

17. Testsystem zum in vitro Nachweis der Aktivierung von T-Zellen enthaltend:
a) mindestens ein T-Zell-Epitop gemäß Anspruch 1 oder 2, mindestens einen Vektor gemäß Anspruch 4, mindestens eine Zelle gemäß einem der Ansprüche 5-8, und/oder mindestens einen Komplex gemäß einem der Ansprüche 9-11, und
b) Effektorzellen des Immunsystems, vorzugsweise T-Zellen, insbesondere zytotoxische T-Zellen oder T-Helferzellen.

18. Verwendung mindestens eines T-Zell-Epitops gemäß Anspruch 1 oder 2, mindestens eines Vektors gemäß Anspruch 4, mindestens einer Zelle gemäß einem der Ansprüche 5-8, und/oder mindestens eines Komplexes gemäß einem der Ansprüche 9-11 zur Herstellung eines Arzneimittels zur Auslösung oder zur Herstellung eines Diagnostikums zum Nachweis einer Immunantwort.

19. Arzneimittel oder Diagnostikum enthaltend mindestens einen Vektor gemäß Anspruch 4, mindestens eine Zelle gemäß einem der Ansprüche 5-8, und/oder mindestens einen Komplex gemäß einem der Ansprüche 9-11 und gegebenenfalls einen pharmazeutisch akzeptablen Träger.

20. Arzneimittel oder Diagnostikum nach Anspruch 19, **dadurch gekennzeichnet, daß** mindestens ein Vektor gemäß Anspruch 4, mindestens eine Zelle gemäß einem der Ansprüche 5-8, und/oder mindestens ein Komplex gemäß einem der Ansprüche 9-11 in Lösung vorliegt, an eine feste Matrix gebunden ist, und/oder mit einem Adjuvans versetzt ist.

21. Verwendung eines T-Zell-Epitops mit einer Aminosäuresequenz AGVDNRECI und/oder eine funktionell aktive Variante davon in vitro als ein zytotoxisches T-Zell-Epitop, wobei die genannte Variante eine Sequenzhomologie zu AGVDNRECI von mindestens 65%, vorzugsweise mindestens 75% und insbesondere mindestens 85% auf Aminosäureebene besitzt.

22. Verwendung eines T-Zell-Epitops mit einer Aminosäuresequenz AGVDNRECI und/oder eine funktionell aktive Variante davon als ein zytotoxisches T-Zell-Epitop zur Herstellung eines Arzneimittels zur Auslösung oder zur Herstellung eines Diagnostikums zum Nachweis einer Immunantwort, wobei die genannte Variante eine Sequenzhomologie zu AGVDNRECI von mindestens 65%, vorzugsweise mindestens 75% und insbesondere mindestens 85% auf Aminosäureebene besitzt.

## Claims

1. T-cell epitope having an amino acid sequence AGVDNRECI.

2. T-cell epitope according to claim 1, **characterised in that** the T-cell epitope is a cytotoxic T-cell epitope.

3. Nucleic acid, **characterised in that** it codes for a T-cell epitope according to claim 1 or 2.

4. Vector, especially an expression vector, **characterised in that** it comprises a nucleic acid according to claim 3.

5. Cell, **characterised in that** it comprises, and preferably presents, at least one T-cell epitope according to claim 1 or 2.

6. Cell according to claim 5, **characterised in that** the cell has been transfected, transformed and/or infected with a nucleic acid according to claim 3 and/or with a vector according to claim 4.

7. Cell according to claim 5, **characterised in that** the cell has been incubated with at least one T-cell epitope according to claim 1 or 2 and/or with at least one complex according to any one of claims 9 to 11.

8. Cell according to claim 5 or 6, **characterised in that** the cell is a B-cell, a macrophage, a dendritic cell, a non-human embryonic cell, a fibroblast, a B16F10 cell, a B6 cell, a C3 cell, an EL4 cell, an RMA cell or an RMA-S cell.

9. Complex comprising a T-cell epitope according to claim 1 or 2 and at least one further compound.

10. Complex according to claim 9, **characterised in that** the complex comprises at least one MHC Class I molecule, preferably as a H2-D^{b} tetramer.

11. Complex according to claim 10, **characterised in that** the said MHC Class I molecule is a human or murine MHC Class I molecule, especially a MHC Class I molecule derived from C57B1/6 mice.

12. Method for the *in vitro* detection of the activation of T-cells by at least one compound, comprising a T-cell epitope according to claim 1 or 2, which method comprises the following steps:
a) stimulation of cells with at least one said compound;
b) addition of at least one target cell that presents a T-cell epitope according to claim 1 or 2 or of a complex according to any one of claims 9 to 11, and
c) determination of the activation of T-cells.

13. Method according to claim 12, **characterised in that** it comprises after step a) the following additional step a'):
a') co-culturing of the cells for about 12 days, especially at least 5 days, with:
(i) at least one target cell charged with a T-cell epitope according to claim 1 or 2, at least one complex according to any one of claims 9 to 11, at least one capsomer, at least one stable capsomer, at least one VLP, at least one CVLP and/or at least one virus,
(ii) at least one complex according to any one of claims 9 to 11,
(iii) and/or at least one target cell that presents a T-cell epitope according to claim 1,
before step b) is carried out.

14. Method of preparing a target cell in accordance with any one of claims 5, 7 and 8, **characterised in that** the target cell is incubated with at least one T-cell epitope according to claim 1 or 2 and/or with at least one complex according to any one of claims 9 to 11 comprising a T-cell epitope according to claim 1 or 2.

15. Method of preparing a target cell in accordance with any one of claims 5, 7 and 8, **characterised in that** the target cell is transfected, transformed and/or infected with a nucleic acid according to claim 3 and/or with a vector according to claim 4.

16. Method of preparing a target cell according to claim 14 or 15, **characterised in that** the target cell is a B-cell, a macrophage, a dendritic cell, a non-human embryonic cell, a fibroblast, a B16F10 cell, a B6 cell, a C3 cell, an EL4 cell, an RMA cell or an RMA-S cell.

17. Test system for *in vitro* detection of the activation of T-cells, comprising:
a) at least one T-cell epitope according to claim 1 or 2, at least one vector according to claim 4, at least one cell according to any one of claims 5 to 8 and/or at least one complex according to any one of claims 9 to 11, and
b) effector cells of the immune system, preferably T-cells, especially cytotoxic T-cells or T-helper cells.

18. Use of at least one T-cell epitope according to claim 1 or 2, at least one vector according to claim 4, at least one cell according to any one of claims 5 to 8 and/or at least one complex according to any one of claims 9 to 11 in the preparation of a medicament for triggering an immune response or in the preparation of a diagnostic agent for detecting an immune response.

19. Medicament or diagnostic agent comprising at least one vector according to claim 4, at least one cell according to any one of claims 5 to 8 and/or at least one complex according to any one of claims 9 to 11 and optionally a pharmaceutically acceptable carrier.

20. Medicament or diagnostic agent according to claim 19, **characterised in that** at least one vector according to claim 4, at least one cell according to any one of claims 5 to 8 and/or at least one complex according to any one of claims 9 to 11 is present in solution, is bonded to a solid matrix and/or is mixed with an adjuvant.

21. Use of a T-cell epitope having an amino acid sequence AGVDNRECI and/or a functionally active variant thereof *in vitro* as a cytotoxic T-cell epitope, the said variant having sequence homology to AGVDNRECI of at least 65%, preferably at least 75% and especially at least 85% at the amino acid level.

22. Use of a T-cell epitope having an amino acid sequence AGVDNRECI and/or a functionally active variant thereof as a cytotoxic T-cell epitope in the preparation of a medicament for triggering an immune response or in the preparation of a diagnostic agent for detecting an immune response, the mentioned variant having sequence homology to AGVDNRECI of at least 65%, preferably at least 75% and especially at least 85% at the amino acid level.

## Revendications

1. Epitope de cellule T ayant une séquence d'acides aminés AGVDNRECI.

2. Epitope de cellule T selon la revendication 1, **caractérisé en ce que** l'épitope de cellule T est un épitope de cellule T cytotoxique.

3. Acide nucléique, **caractérisé en ce qu'**il code pour un épitope de cellule T selon la revendication 1 ou la revendication 2.

4. Vecteur, en particulier vecteur d'expression, **caractérisé en ce qu'**il contient un acide nucléique selon la revendication 3.

5. Cellule, **caractérisée en ce qu'**elle contient, de préférence présente, au moins un épitope de cellule T selon la revendication 1 ou la revendication 2.

6. Cellule selon la revendication 5, **caractérisée en ce que** la cellule est transfectée, transformée et/ou infectée avec un acide nucléique selon la revendication 3 et/ou avec un vecteur selon la revendication 4.

7. Cellule selon la revendication 5, **caractérisée en ce que** la cellule a été incubée avec au moins un épitope de cellule T selon la revendication 1 ou la revendication 2 et/ou avec au moins un complexe selon l'une des revendications 9 à 11.

8. Cellule selon la revendication 5 ou la revendication 6, **caractérisée en ce que** la cellule est une cellule B, un macrophage, une cellule dendritique, une cellule embryonnaire non humaine, un fibroblaste, une cellule B16F10, B6, C3, EL4, RMA ou RMA-S.

9. Complexe contenant un épitope de cellule T selon la revendication 1 ou la revendication 2 et au moins un autre composé.

10. Complexe selon la revendication 9, **caractérisé en ce que** le complexe contient au moins une molécule de CMH de classe 1, de préférence sous la forme d'un tétramère H2-D^{b}.

11. Complexe selon la revendication 10, **caractérisé en ce que** ladite molécule de CMH de classe 1 est une molécule de CMH de classe 1 humaine ou murine, en particulier une molécule de CMH de classe 1 provenant de souris C57B1/6.

12. Procédé de détermination *in vitro* de l'activation de cellules T par au moins un composé contenant un épitope de cellule T selon la revendication 1 ou la revendication 2, ledit procédé comprenant les étapes consistant à :
a) stimuler les cellules avec l'un au moins des composés précités ;
b) ajouter au moins une cellule cible qui présente un épitope de cellule T selon la revendication 1 ou la revendication 2 ou bien un complexe selon l'une des revendications 9 à 11, et
c) déterminer l'activation des cellules T.

13. Procédé selon la revendication 12, **caractérisé en ce que**, après l'étape a), il contient l'étape supplémentaire a') consistant à :
a) a') co-cultiver les cellules pendant environ 12 jours, en particulier pendant au moins 5 jours, avec:
(i) au moins une cellule cible chargée avec un épitope de cellule T selon la revendication 1 ou la revendication 2, au moins un complexe selon l'une des revendications 9 à 11, au moins un capsomère, au moins un capsomère stable, au moins une pseudo-particule virale (VLP), au moins une pseudo-particule virale chimérique (CVLP) et/ou au moins un virus,
(ii) au moins un complexe selon l'une des revendications 9 à 11,
(iii) et/ou au moins une cellule cible qui présente un épitope de cellule T selon la revendication 1,
avant de passer à l'étape b).

14. Procédé de production d'une cellule cible selon l'une des revendications 5, 7 ou 8, **caractérisé en ce que** la cellule cible est incubée avec au moins un épitope de cellule T selon la revendication 1 ou la revendication 2 et/ou avec au moins un complexe selon l'une des revendications 9 à 11 contenant un épitope de cellule T selon la revendication 1 ou la revendication 2.

15. Procédé de production d'une cellule cible selon l'une des revendications 5, 7 ou 8, **caractérisé en ce que** la cellule cible est transfectée, transformée et/ou infectée avec un acide nucléique selon la revendication 3 et/ou un vecteur selon la revendication 4.

16. Procédé de production d'une cellule cible selon la revendication 14 ou la revendication 15, **caractérisé en ce que** la cellule cible est une cellule B, un macrophage, une cellule dendritique, une cellule embryonnaire non humaine, un fibroblaste, une cellule B16F10, B6, C3, EL4, RMA ou RMA-S.

17. Système de test pour la détermination *in vitro* de l'activation de cellules T, contenant :
a) au moins un épitope de cellule T selon la revendication 1 ou la revendication 2, au moins un vecteur selon la revendication 4, au moins une cellule selon l'une des revendications 5 à 8 et/ou au moins un complexe selon l'une des revendications 9 à 11, et
b) des cellules effectrices du système immunitaire, de préférence des cellules T, en particulier des cellules T cytotoxiques ou des cellules T auxiliaires.

18. Utilisation d'au moins un épitope de cellule T selon la revendication 1 ou la revendication 2, d'au moins un vecteur selon la revendication 4, d'au moins une cellule selon l'une des revendications 5 à 8 et/ou d'au moins un complexe selon l'une des revendications 9 à 11 pour préparer un médicament destiné à déclencher une réponse immunitaire ou pour préparer un produit de diagnostic permettant de détecter une réponse immunitaire.

19. Médicament ou produit de diagnostic contenant au moins un vecteur selon la revendication 4, au moins une cellule selon l'une des revendications 5 à 8 et/ou au moins un complexe selon l'une des revendications 9 à 11 ainsi que, le cas échéant, un véhicule pharmaceutiquement acceptable.

20. Médicament ou produit de diagnostic selon la revendication 19, **caractérisé en ce qu'**au moins un vecteur selon la revendication 4, au moins une cellule selon l'une des revendications 5 à 8 et/ou au moins un complexe selon l'une des revendications 9 à 11 est présent en solution, lié à une matrice solide et/ou mélangé avec un adjuvant.

21. Utilisation d'un épitope de cellule T ayant une séquence d'acides aminés AGVDNRECI et/ou d'une variante de celui-ci fonctionnellement active *in vitro* comme épitope de cellule T cytotoxique, ladite variante ayant avec AGVDNRECI une homologie de séquence d'au moins 65%, de préférence au moins 75% et en particulier au moins 85% au niveau des acides aminés.

22. Utilisation d'un épitope de cellule T ayant une séquence d'acides aminés AGVDNRECI et/ou d'une variante de celui-ci fonctionnellement active comme épitope de cellule T cytotoxique pour préparer un médicament destiné à déclencher une réponse immunitaire ou pour préparer un produit de diagnostic permettant de détecter une réponse immunitaire, ladite variante ayant avec AGVDNRECI une homologie de séquence d'au moins 65%, de préférence au moins 75% et en particulier au moins 85% au niveau des acides aminés.
